# EUROPEAN PATENT SPECIFICATION

(11) **EP 4 593 684 B1**
(45) Date of publication and mention of the grant of the patent: **11.11.2026**
(21) Application number: 23786330.3
(22) Date of filing: 27.09.2023
(51) Int. Cl.: A61B 3/00, A61B 3/10, A61F 9/008

(54) **VITREOUS FLOATER CHARACTERIZATION USING ABERROMETRY**
CHARAKTERISIERUNG VON GLASKÖRPERN MITTELS ABERROMETRIE
CARACTÉRISATION DE CORPS FLOTTANT VITRÉEN À L'AIDE D'UNE ABERROMÉTRIE

(30) Priority: 27.09.2022 US 202263377223 P
(43) Date of publication of application: 06.08.2025
(73) Proprietor: Alcon Inc., 1701 Fribourg (CH)
(72) Inventor: HALLEN, Paul R, Fort Worth, Texas 76134 (US)
(74) Representative: Murgitroyd & Company
(86) International application number: PCT/IB2023/059629
(87) International publication number: WO 2024/069485

(56) References cited:
- WO-A1-2022/149028
- US-A1- 2009 073 384
- US-A1- 2010 123 873
- US-A1- 2014 257 257

## Description

### BACKGROUND

The posterior chamber of the eye between the crystalline lens and the retina is occupied by a transparent gel known as the vitreous. Due to various causes, floaters may be present in the vitreous. Floaters are typically formed of clumps of cells and may have varying sizes and opacities. All eyes will have some floaters. However, the size, number, and opacity of floaters in the eye may be such that vision is significantly impaired. In that case, treatment may include removing the vitreous and replacing it with saline or a bubble made of gas or oil. Reference is made to the documents US 2010/123873 A1, US 2009/073384 A1, WO 2022/149028 A1 and US 2014/257257 A1 which have been cited as exemplary of the state of the art. US 2010/123873 A1 relates to a method and system for qualifying light spots for use for optical measurements by a measurement instrument, and based on light spot data provided by a wavefront sensor.

### BRIEF SUMMARY

The scope of the invention is in accordance with the appended claims.

The present disclosure relates generally to a system for characterizing vitreous floaters using aberrometry.

In one aspect, a method, not claimed in the appended claims, performed by a computing device includes receiving one or more wavefront elevation maps for a patient's eye. The wavefront elevation maps are processed to identify one or more attributes of the one or more wavefront elevation maps corresponding to vitreous floaters. The one or more attributes may include localized spatial variation of the one or more wavefront elevation maps; temporal variation among a plurality of wavefront elevation maps; and depth information indicating scattering of light from within the vitreous of the patient's eye.

The following description and the related drawings set forth in detail certain illustrative features of one or more embodiments.

### BRIEF DESCRIPTION OF THE DRAWINGS

The appended figures depict certain aspects of the one or more embodiments and are therefore not to be considered limiting of the scope of this disclosure.
Fig. 1 is a schematic block diagram of a system for floater characterization using aberrometry, in accordance with certain embodiments.
Fig. 2 is a schematic block diagram of a system for performing aberrometery along with depth measurement of scattered light, in accordance with certain embodiments.
Fig. 3 is a process flow diagram of a method for characterizing floaters using aberrometry, in accordance with certain embodiments.
Fig. 4 is a process flow diagram of a method for training a machine learning model to characterize floaters using aberrometery data, in accordance with certain embodiments.
Fig. 5 illustrates an example computing device that implements, at least partly, one or more functionalities of creating and presenting a primary treatment plan and one or more backup plans.

To facilitate understanding, identical reference numerals have been used, where possible, to designate identical elements that are common to the drawings. It is contemplated that elements and features of one embodiment may be beneficially incorporated in other embodiments without further recitation.

### DETAILED DESCRIPTION

Aberrometery is a relatively inexpensive approach for characterizing the refractive error of the eye. The output of an aberrometer is a wavefront elevation map in which the elevation at each point indicates a phase delay of a point on a planar wavefront passing through the cornea, lens, and vitreous of the eye to the retina. The wavefront elevation map may then be analyzed to characterize the refractive error of the eye. Using the approach described herein, an aberrometer is used to characterize the clinical significance of floaters in the eye.

A system 100 includes an aberrometer 102 configured to detect the refractive error of an eye 110. The aberrometer 102 may implement wavefront aberrometry that measures the propagation of a wavefront through the eye 110. In particular, the aberrometer 102 measures the propagation of the wavefront through the cornea 112, crystalline lens 114, capsulary bag 116, and vitreous 118 to the retina 120. For example, the aberrometer 102 may be implemented as or similar to the Optiwave Refractive Analysis (ORA) system by ALCON. The output of the aberrometer 102 is a wavefront elevation map comprising a two-dimensional array of values in which each value represents a point from which light was scattered and the elevation at each point represents the phase delay of a wavefront upon incident on that point. In some implementations, a two-dimensional array of amplitude of values such that for each index (e.g., X, Y coordinate) a phase and an amplitude value are stored indicating the phase delay and amplitude of light scattered from a point in space corresponding to the X, Y coordinate. The light beams emitted by the aberrometer 102 will be scattered by any floaters 122 within the vitreous. The degree of scattering will correspond to the number of floaters 122, the size of the floaters 122, and the opacity of the floaters 122.

The aberrometer 102 may be used alone to characterize floaters 122 or may be used in combination with other ophthalmic measurement devices such as a scanning laser ophthalmoscope (SLO) 104 and/or optical coherence tomography (OCT) device 106. For example, a SLO 104 and/or OCT device 106 may be used to confirm the presence of floaters 122 detected using the aberrometer 102 as described below and/or to precisely identify the locations of any detected floaters 122. In some implementations, the aberrometer 102, SLO 104, and OCT device 106 are contained within a same housing.

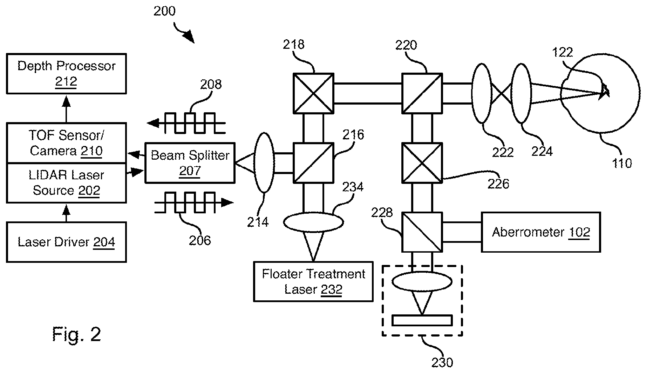

Referring to Fig. 2, an aberrometer 102 may be used as part of the illustrated system 200 in order to detect floaters 122. The system 200 includes a light detection and ranging (LIDAR) laser source 202 controlled by a laser driver 204, which causes the LIDAR laser source 202 to emit a series of pulses 206 having a known period between pulses and duration of pulses. The pulses 206 are pass through a beam splitter 207 to the eye 110. Return pulses 208 including light reflected from the eye 110 pass through the beam splitter and a portion thereof is received by a time-of-flight (TOF) sensor/camera 210. TOF measurements from the TOF sensor/camera 210 are input to a depth processor 212, which interprets the TOF measurements to estimate depth of a point in the eye 110 that scattered the return pulses 208.

The pulses 206 may reach the eye 110 by passing through a collimating lens 402 and a dichroic mirror 216, which directs light from the collimating lens 402 onto a rotating mirror 218 rotating about one or two axes, such as a galvo scanner. Light reflected by the rotating mirror 218 is incident on a dichroic mirror 220. The portion of light transmitted through the dichroic mirror is incident on one or more objective lenses 222, 224 that focus the light output from the objective lenses onto the eye 110. Light scattered by the eye 110, including floaters 122 within the eye 110, returns through the objective lenses 222, 224, transmits through the dichroic mirror 220, is descanned by the rotating mirror 2187, is incident on dichroic mirror 216, passes through the lens 214, and is incident on the beam splitter 1010. A portion of the light scattered by the eye 110 is directed by the beam splitter 1010 onto the TOF sensor/camera 210.

A portion of the light scattered by the eye 110 is directed by the dichroic mirror 220 onto another rotating mirror 226, which may be rotatable about one or two axes and be implemented as a galvo mirror. The rotating mirror 226 descans the portion of the scattered light onto a dichroic mirror 228, which directs a portion of the descanned light onto the aberrometer 102. A portion of the descanned light is transmitted through the dichroic mirror onto a fixation target used to calibrate the TOF sensor/camera 210.

In the illustrated embodiment, a floater treatment laser 232 is also included. For example, light from the floater treatment laser 232 may pass through a collimating lens 234, through the dichroic mirror 216, onto the rotating mirror 218, which scans the light from the floater treatment laser 232 onto the eye 110 through the dichroic mirror 220 and objective lenses 222, 224 onto the eye 110. The floater treatment laser 232 may generate pulses of light focused on floaters 122 and having sufficient intensity to disintegrate floaters 122. The timing and depth of focus of pulses generated by the floater treatment laser 232 may be controlled using locations and depth of floaters 122 determined using the TOF sensor/camera 210 and depth processor 212. For example, a depth of focus of a treatment pulse from the floater treatment laser 232 may be selected using a depth estimate from the depth processor 212 and the treatment pulse may be emitted when the rotating mirror 218 is at the same position as when the pulse 206 was emitted that was used to obtain the depth estimate.

In the foregoing description, various beam splitters and dichroic mirrors are discussed. It shall be understood that the arrangements of elements as receiving transmitted or reflected light from a beam splitter or dichroic mirror may be reversed: a first element receiving transmitted light and a second element receiving reflected light from a beam splitter or dichroic mirror may be substituted with the first element receiving the reflected light and the second element receiving the transmitted light.

The system 200 uses the LIDAR laser source 202 to bounce low-power optical pulses off a target, i.e., the eye 110, and detecting the light reflected using the TOF sensor/camera 210. The TOF sensor/camera 210 captures time-stamped images. The depth processor 212 then calculates a time of flight based on the time-stamp and the known transmission time of each pulse 206. The distance to a structure that scattered light detected in an image may then be calculated using the speed of light. The distance may be combined with the known orientation of the rotating mirror 218 to obtain high resolution information in all three dimensions. In particular, for each pulse 208 received, the position of the rotating mirror 218 and distance estimate may be used to obtain a three dimensional coordinate of a point in the eye 110 that scattered the pulse 208. The amplitude of each pulse 208 indicates the reflectivity (and correspondingly the opacity) of the point in the eye. Accordingly, a point cloud results in which each point has a three dimensional coordinate and a reflectivity.

The LIDAR laser source 202 can emit hundreds of thousands of pulses 206 per second. Some of the light from each pulse may be reflected from a floater 122 returned to the TOF sensor/camera 210. The point cloud would therefore include points corresponding to pulses 206 reflected from the floater 122. Due to the small size of floaters 122, a resolution of less than 100 mm is desired, which may be obtained using a pulse generation frequency of more than 350 MHz.

The aberrometer 102 measures wavefront aberrations of the eye, such as by using reflected light from the same pulses 206. The floaters 122 inside the eye, which may be moving, act as disturbance to the optical path. The floaters 122 cause fluctuations in the index of refraction along the beam path and scatter light, both of which add noise to the wavefront measurement. By performing TOF and wavefront measurements simultaneously, the wavefront deviations along different sections of the beam path through the vitreous 118 may be correlated to the location of a structure, e.g., floater 122, that caused the wavefront deviation. The aberrometer 102 may also provide a continuous sampling of the pupil size. The 3D measurements may further be used with tomographic reconstruction software to generate refractive map of vitreous 118. Such a refractive map indicates the locations, size, and opacity of floaters 122. Once floaters 122 have been identified as described above, the clinical significance of the floaters may be determined using the following parameters. For example, the distance between the floaters and retina, the area and darkness of the shadow caused by the floaters on retain etc. Magnitude of disturbance of the floaters can be estimated using local variance (or roughness) of the waveform profile.

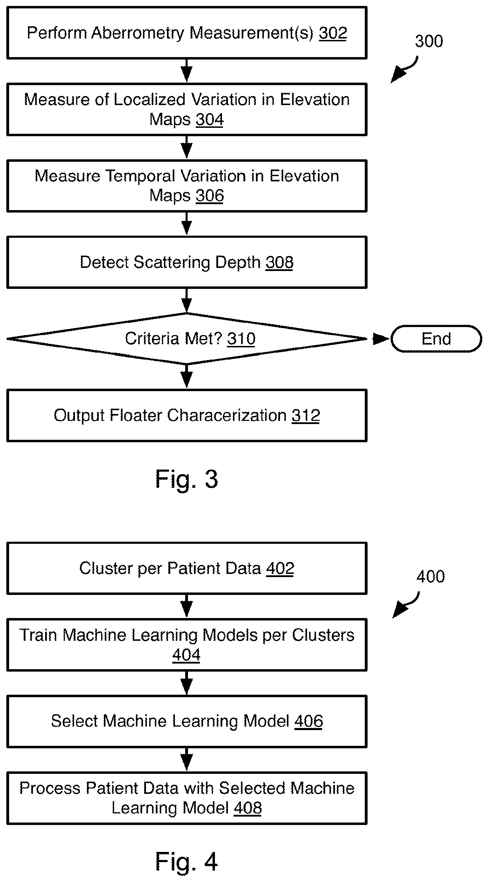

Fig. 3 illustrates a method 300, not claimed in the appended claims, for characterizing floaters using aberrometery. The method 300 may be performed by the floater detection module 108 using wavefront elevation maps received from the aberrometer 102. The method 300 may be performed with or without the system 200 as described in greater detail below. The method 300 identifies attributes of one or more wavefront elevation maps that may correspond to vitreous floaters in order to determine the clinical significance of the vitreous floaters. These attributes include some or all of localized spatial variation, temporal variation, and depth values as described in greater detail below.

The method 300 includes performing, at step 302, aberrometery measurements of the patient's eye 110. Step 302 may include simply performing multiple aberrometery measurements at a fixed or variable period. Step 302 may include performing aberrometry measurements simultaneously with distance measurements as described above with respect to the system 200. The result of each aberrometery measurement is a wavefront elevation map. The wavefront elevation map may be viewed as an image in which each pixel position (X, Y coordinate) has an elevation value associated therewith indicating a phase delay. As noted above, each X, Y coordinate may also have an amplitude value. The wavefront elevation map will have a generally circular shape corresponding to the pupil of the eye 110.

The method 300 may include measuring, at step 304, localized variation in each wavefront elevation map. Refractive error in the eye 110 will result in the wavefront elevation map being non-planar. The presence of cataracts likewise results in scattering that is uniformly distributed. However, the scattering caused by a floater 122 may result in localized elevation variation in a wavefront elevation map that is distinguishable from elevation variation caused by refractive error. Localized variation may be measured in various ways. For example, a sliding-window two-dimensional spatial Fourier transform may be calculated for each elevation map or for discrete regions at some or all points (e.g., X, Y coordinates) in each selected elevation map for one or both of phase and amplitude values. For example, the discrete regions may have height and width that is between 0.05 and 0.15 times the diameter of the wavefront elevation map. Frequencies above a frequency threshold and with a magnitude above a magnitude threshold in a Fourier transform may be deemed to correspond to vitreous floaters. The output of step 304 may therefore be a function of the magnitude (e.g., average, maximum, integral) of the portion of the two-dimensional Fourier transform above a threshold frequency. There are many other methods to measure and analyze local variation of the wavefront (phase elevation map), such as Landmark-based geometric morphometrics, principal components analysis, as well as analyses of global variational properties of morphological features, such as morphological integration etc. Also, wavefronts acquired over a period of time can be analyzed to obtain the de-correlation of wavefront change/fluctuation over time. The de-correlation of the wavefront change/fluctuation over time can be analyzed to determine which area of the phase elevation map fluctuates the most due to the floaters.

The method 300 may include measuring, at step 306, temporal variation among the wavefront elevation maps. Floaters 122 may move within the vitreous 118 such that any scattering caused thereby is different in wavefront elevation maps captured at different times. In contrast, variation in wavefront elevation maps caused by cataracts or refractive error should not change over time. Step 306 may be accompanied by performing eye tracking and compensating for eye movement when performing aberrometry measurements at step 302. In this manner, movement in the locations of regions of localized variation can more reasonably be attributed to movement of floaters 122 rather than eye movement. Measuring temporal variation may be performed in various ways. For example, a Z coordinate may be defined as an index assigned to each elevation map, with the indexes corresponding to the temporal ordering in which the wavefront elevation maps were obtained at step 302. Temporal variation may therefore be obtained by characterizing variation along the Z axis. For example, for a given X, Y coordinate a volume may be defined including the X, Y coordinates and the range of possible Z coordinates. A three-dimensional Fourier transform of this volume may therefore be calculated for this volume at step 302 for one or both of phase and amplitude values. As an alternative, an alternative volume may be defined as the Fourier transforms from step 304 for all Z coordinates. Values of either three-dimensional Fourier transform at frequencies above a frequency threshold and with a magnitude above a magnitude threshold may be deemed to correspond to vitreous floaters. The output of step 306 may therefore be a function of the magnitude (e.g., average, maximum, integral) of the three-dimensional Fourier transform above a threshold frequency.

In the case where the system 200 is used, the method 300 may further include measuring, at step 308, the scattering depth, such as using TOF measurements as described above. For example, scattering found at a depth that is between the capsular bag 116 and the retina 120, i.e., the vitreous 118, may be deemed to correspond to vitreous floaters 122. Step 308 may be omitted in order to characterize floaters using only an aberrometer 102. Step 308 may include measuring the reflectivity of points within the vitreous 118 and generating a score based thereon, e.g. a sum or weighted sum of the reflectivity of points within the vitreous 118.

Step 308 may also be performed using another imaging modality such an SLO 104 or OCT device 106. An SLO 104 is capable of measuring the distance of a floater to the retina by focusing on the retina and then shifting the SLO focus to the floater. The distance to the retina can be measured by converting focus diopter change to a distance. An OCT device 106 is also capable of measuring distance of floater to ocular structures in a like manner. Either a long-range OCT can be used to detect the full vitreous in one scan or a shorter-range OCT can be used with multiple scans to detect floaters in the vitreous. The distance to the retina can be measured with proper calibration of the OCT system and by converting pixel locations to distance measurements.

The method 300 may include evaluating, at step 310, whether a clinical criteria has been met. For example, some or all of the localized variation as measured at step 304, the temporal variation as measured at step 306, and the scattering depth as measured at step 308 may be evaluated with respect to individual thresholds or combined (e.g., summed, weighted and summed, multiplied, etc.) and compared to a single threshold. Where some or all of the individual thresholds are exceeded or the single threshold is exceeded, the patient's eye 110 may be deemed to have clinically significant floaters such that laser ablation or vitrectomy is needed. Alternatively or additionally, where the criteria of step 310 are met, other actions may be performed, such as scanning the eye 110 using another imaging modality, such as an SLO 104 or OCT device 106 in order to map the size and location of the vitreous floaters.

If the clinical criteria is found to have been met, then a characterization of vitreous floaters may be output at step 312, which may include any of the information evaluated at step 310 (localized variation as measured at step 304, the temporal variation as measured at step 306, and/or the scattering depth as measured at step 308).

Fig. 4 illustrates a method 400, not claimed in the appended claims, that may be used to characterize vitreous floaters. The method 400 may be used with, or in place of, the method 300. For example, the method 400 may be used at step 310 to determine whether clinically significant floaters are present.

The method 400 may include clustering, at step 402, training data entries. Training data entries may be clustered using k-means clustering, k nearest neighbor, or other clustering technique. Training entries may include, as inputs, patient data including such information as demographic data (age, sex, ethnicity) and comorbidities (cataracts, retinal disease). The inputs of each entry further includes aberrometry data in the form of a single wavefront elevation map or a series of wavefront elevation maps obtained over time as described above with respect to step 302. Each wavefront elevation map may include one or both of phase and amplitude values. Each training data entry may further include, as a desired output, a metric of floaters detected in the eye of the patient, such as obtained using an SLO or OCT device. The metric of floaters may include a human generated estimate of clinical significance, a score indicating combined reflectivity of points within the vitreous 118, or some other metric of floaters. Clustering at step 402 may include performing multiple linear regression with the inputs of the training data entries as explanatory data and the desired output of the training data entries as the response variable.

The training data entries of each cluster may then be used to train a machine learning model for each cluster such that each cluster has a corresponding machine learning model. Each machine learning model may be trained to output, for a given set of inputs, an estimate of clinical significance of vitreous floaters. Each machine learning model may be any machine learning model known in the art such as a deep neural network (DNN), convolution neural network (CNN), multiple polynomial regression (MPR) (2^{nd} order, 3^{rd} order, or higher) model, support vector regression model (SVM), or SVM radial bias function (SVM-RBF).

During utilization, patient data (demographic data, co-morbidities, wavefront elevation map(s)) may be processed to select, at step 406, a corresponding cluster, such as using the same clustering technique used at step 402 or a different clustering technique. The patient data may then be processed, at step 408, using the machine learning model for the selected cluster to obtain a predicted clinical significance of floaters in the patient's eye. The utilization of steps 406 and 408 may be performed using a different computing device than is used to perform steps 402 and 404.

The method 400 is exemplary only. For example, clustering 402 may be omitted and a single machine learning model trained to predict clinical significance of floaters using the training patient data. The training patient data used for each entry may include less than or more than the items of data listed above. For example, comorbidities may be omitted and the machine learning model trained to predict clinical significance of floaters without *a priori* knowledge of co-morbidities, such as cataracts or retinal disease. In particular, cataracts will scatter light used to perform aberrometery and the machine learning model (or models) may be trained to account for this phenomenon.

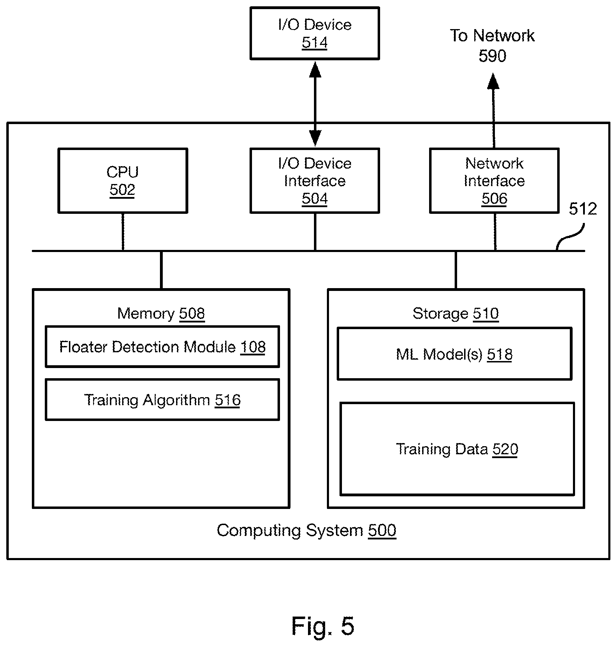

Fig. 5 illustrates an example computing system 500, not claimed in the appended claims, that implements, at least partly,
one or more functionalities described, such as one or both of the method 300, the method 400, and processing described above with respect to the system 200. The computing system 500 may be integrated with an imaging device, such as an aberrometer 102, or be a separate computing device receiving images of a patient's eye from the imaging device.

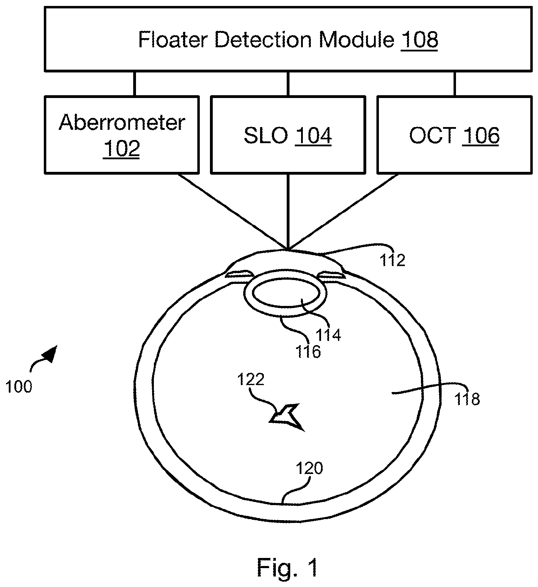

As shown, computing system 500 includes a central processing unit (CPU) 502, one or more I/O device interfaces 504, which may allow for the connection of various I/O devices 514 (e.g., keyboards, displays, mouse devices, pen input, etc.) to computing system 500, network interface 506 through which computing system 500 is connected to network 590 (which may be a local network, an intranet, the internet, or any other group of computing systems communicatively connected to each other, as described in relation to FIG. 1), a memory 508, storage 510, and an interconnect 512.

In cases where computing system 500 is an imaging system, such as a digital microscope, computing system 500 may further include one or more optical components for obtaining ophthalmic imaging of a patient's eye as well as any other components known to one of ordinary skill in the art. In cases where computing system 500 is a surgical microscope, computing system 500 may further include many other components known to one of ordinary skill in the art to perform the ophthalmic surgeries described herein as known to one of ordinary skill in the art.

CPU 502 may retrieve and execute programming instructions stored in the memory 508. Similarly, CPU 502 may retrieve and store application data residing in the memory 508. The interconnect 512 transmits programming instructions and application data, among CPU 502, I/O device interface 504, network interface 506, memory 508, and storage 510. CPU 502 is included to be representative of a single CPU, multiple CPUs, a single CPU having multiple processing cores, and the like.

Memory 508 is representative of a volatile memory, such as a random access memory, and/or a nonvolatile memory, such as nonvolatile random access memory, phase change random access memory, or the like. As shown, memory 508 may store executable code that is executable by the CPU 502 to implement some or all of the floater detection module 108, the processing described above with respect to the system 200, and the method 300. The memory 508 may additionally or alternatively store executable code implementing a training algorithm 516 used during steps 402 and 404 of the method 400.

Storage 510 may be non-volatile memory, such as a disk drive, solid state drive, or a collection of storage devices distributed across multiple storage systems. Storage 510 may optionally store a machine learning model 518, or multiple machine learning models 518, trained as described above with respect to Fig. 4. Where the computing system 500 is used for training the machine learning model(s) 514, the storage 510 may further store training data 520 including a plurality of training data entries including patient data as described above with respect to Fig. 4.

### Additional Considerations

The scope of the invention is in accordance with the appended claims. The preceding description is provided to enable any person skilled in the art to practice the various embodiments described herein. Various modifications to these embodiments will be readily apparent to those skilled in the art, and the generic principles defined herein may be applied to other embodiments.

As used herein, a phrase referring to "at least one of" a list of items refers to any combination of those items, including single members. As an example, "at least one of: a, b, or c" is intended to cover a, b, c, a-b, a-c, b-c, and a-b-c, as well as any combination with multiples of the same element (e.g., a-a, a-a-a, a-a-b, a-a-c, a-b-b, a-c-c, b-b, b-b-b, b-b-c, c-c, and c-c-c or any other ordering of a, b, and c).

As used herein, the term "determining" encompasses a wide variety of actions. For example, "determining" may include calculating, computing, processing, deriving, investigating, looking up (e.g., looking up in a table, a database or another data structure), ascertaining and the like. Also, "determining" may include receiving (e.g., receiving information), accessing (e.g., accessing data in a memory) and the like. Also, "determining" may include resolving, selecting, choosing, establishing and the like.

The scope of the invention is in accordance with the appended claims. The methods disclosed herein, not claimed in the appended claims,
comprise one or more steps or actions for achieving the methods. The method steps and/or actions may be interchanged with one another.

Further, the various operations of methods described above may be performed by any suitable means capable of performing the corresponding functions. The means may include various hardware and/or software component(s) and/or module(s), including, but not limited to a circuit, an application specific integrated circuit (ASIC), or processor. Generally, where there are operations illustrated in figures, those operations may have corresponding counterpart means-plus-function components with similar numbering.

The various illustrative logical blocks, modules and circuits described in connection with the present disclosure may be implemented or performed with a general purpose processor, a digital signal processor (DSP), an application specific integrated circuit (ASIC), a field programmable gate array (FPGA) or other programmable logic device (PLD), discrete gate or transistor logic, discrete hardware components, or any combination thereof designed to perform the functions described herein. A general-purpose processor may be a microprocessor, but in the alternative, the processor may be any commercially available processor, controller, microcontroller, or state machine. A processor may also be implemented as a combination of computing devices, e.g., a combination of a DSP and a microprocessor, a plurality of microprocessors, one or more microprocessors in conjunction with a DSP core, or any other such configuration.

A processing system may be implemented with a bus architecture. The bus may include any number of interconnecting buses and bridges depending on the specific application of the processing system and the overall design constraints. The bus may link together various circuits including a processor, machine-readable media, and input/output devices, among others. A user interface (e.g., keypad, display, mouse, joystick, etc.) may also be connected to the bus. The bus may also link various other circuits such as timing sources, peripherals, voltage regulators, power management circuits, and the like, which are well known in the art, and therefore, will not be described any further. The processor may be implemented with one or more general-purpose and/or special-purpose processors. Examples include microprocessors, microcontrollers, DSP processors, and other circuitry that can execute software. Those skilled in the art will recognize how best to implement the described functionality for the processing system depending on the particular application and the overall design constraints imposed on the overall system.

If implemented in software, the functions may be stored or transmitted over as one or more instructions or code on a computer-readable medium. Software shall be construed broadly to mean instructions, data, or any combination thereof, whether referred to as software, firmware, middleware, microcode, hardware description language, or otherwise. Computer-readable media include both computer storage media and communication media, such as any medium that facilitates transfer of a computer program from one place to another. The processor may be responsible for managing the bus and general processing, including the execution of software modules stored on the computer-readable storage media. A computer-readable storage medium may be coupled to a processor such that the processor can read information from, and write information to, the storage medium. In the alternative, the storage medium may be integral to the processor. By way of example, the computer-readable media may include a transmission line, a carrier wave modulated by data, and/or a computer readable storage medium with instructions stored thereon separate from the wireless node, all of which may be accessed by the processor through the bus interface. Alternatively, or in addition, the computer-readable media, or any portion thereof, may be integrated into the processor, such as the case may be with cache and/or general register files. Examples of machine-readable storage media may include, by way of example, RAM (Random Access Memory), flash memory, ROM (Read Only Memory), PROM (Programmable Read-Only Memory), EPROM (Erasable Programmable Read-Only Memory), EEPROM (Electrically Erasable Programmable Read-Only Memory), registers, magnetic disks, optical disks, hard drives, or any other suitable storage medium, or any combination thereof. The machine-readable media may be embodied in a computer-program product.

A software module may comprise a single instruction, or many instructions, and may be distributed over several different code segments, among different programs, and across multiple storage media. The computer-readable media may comprise a number of software modules. The software modules include instructions that, when executed by an apparatus such as a processor, cause the processing system to perform various functions. The software modules may include a transmission module and a receiving module. Each software module may reside in a single storage device or be distributed across multiple storage devices. By way of example, a software module may be loaded into RAM from a hard drive when a triggering event occurs. During execution of the software module, the processor may load some of the instructions into cache to increase access speed. One or more cache lines may then be loaded into a general register file for execution by the processor. When referring to the functionality of a software module, it will be understood that such functionality is implemented by the processor when executing instructions from that software module.

The scope of the invention is in accordance with the appended claims.

## Claims

1. An ophthalmic system (100) for guiding ophthalmic surgery comprising:
an aberrometer (102);
one or more processing devices (502);
one or more memory devices (508) coupled to the one or more processing devices and storing executable code that, when executed by the one or more processing devices, causes the one or more processing devices to:
receive one or more wavefront elevation maps for a patient's eye (110) from the aberrometer (102); **characterised in that** the executable code, when executed by the one or more processing devices, causes the one or more processing devices to identify one or more attributes of the one or more wavefront elevation maps corresponding to vitreous floaters (122).

2. The ophthalmic system (100) of claim 1, wherein the one or more attributes include localized spatial variation of the one or more wavefront elevation maps.

3. The ophthalmic system (100) of claim 1, wherein the one or more wavefront elevation maps comprise a plurality of wavefront elevation maps captured at different times and the one or more attributes include temporal variation among the plurality of wavefront elevation maps.

4. The ophthalmic system (100) of claim 1, wherein the one or more wavefront elevation maps comprise a plurality of wavefront elevation maps captured at different times and the one or more attributes include temporal variation among the plurality of wavefront elevation maps and localized spatial variation of the plurality of wavefront elevation maps.

5. The ophthalmic system (100) of claim 1, wherein the aberrometer (102) is arranged according to one or more of the following:
(i) wherein the aberrometer (102) is configured to detect the refractive error of an eye (110);
(ii) wherein the aberrometer (102) is configured to implement wavefront aberrometry that measures the propagation of a wavefront through the eye (110);
wherein the aberrometer (102) is configured such that the output of the aberrometer (102) is a wavefront elevation map comprising a two-dimensional array of values in which each value represents a point from which light was scattered and the elevation at each point represents the phase delay of a wavefront upon incident on that point.

6. The ophthalmic system (100) of claim 1, further comprising:
a light distancing and ranging, LIDAR, system (200) combined with the aberrometer (102) and configured to capture depth information simultaneously with the one or more wavefront elevation maps;
wherein the one or more attributes include the depth information.

7. The ophthalmic system (100) of claim 6, wherein the one or more attributes include whether the depth information indicates scattering within a vitreous (118) of the patient's eye (110).

8. The ophthalmic system (100) of claim 6, wherein the aberrometer (102) and the LIDAR system (200) have a common laser light source (202) and a common scanning mirror (218).

9. The ophthalmic system (100) of claim 1, wherein the executable code that, when executed by the one or more processing devices (502), further causes the one or more processing devices to identify the one or more attributes of the one or more wavefront elevation maps corresponding to the vitreous floaters (122) by processing the one or more wavefront elevation maps with a machine learning model (518).

## Patentansprüche

1. Ophthalmisches System (100) zum Führen von ophthalmischer Chirurgie, umfassend:
ein Aberrometer (102);
eine oder mehrere Verarbeitungsvorrichtungen (502);
eine oder mehrere Speichervorrichtungen (508), die an die eine oder mehreren Verarbeitungsvorrichtungen gekoppelt sind und ausführbaren Code speichern, der bei Ausführung durch die eine oder mehreren Verarbeitungsvorrichtungen die eine oder mehreren Verarbeitungsvorrichtungen veranlasst zum:
Empfangen einer oder mehrerer Wellenfront-Höhenkarten für das Auge eines Patienten (110) von dem Aberrometer (102); **dadurch gekennzeichnet, dass** der ausführbare Code bei Ausführung durch die eine oder mehreren Verarbeitungsvorrichtungen die eine oder mehreren Verarbeitungsvorrichtungen veranlasst zum Identifizieren eines oder mehrerer Attribute der einen oder mehreren Wellenfront-Höhenkarten, die Glaskörpertrübungen entsprechen (122).

2. Ophthalmisches System (100) nach Anspruch 1, wobei das eine oder die mehreren Attribute eine lokalisierte räumliche Variation der einen oder mehreren Wellenfront-Höhenkarten umfassen.

3. Ophthalmisches System (100) nach Anspruch 1, wobei die eine oder mehreren Wellenfront-Höhenkarten mehrere Wellenfront-Höhenkarten umfassen, die zu unterschiedlichen Zeiten erfasst werden, und das eine oder die mehreren Attribute eine zeitliche Variation zwischen den mehreren Wellenfront-Höhenkarten umfassen.

4. Ophthalmisches System (100) nach Anspruch 1, wobei die eine oder mehreren Wellenfront-Höhenkarten mehrere Wellenfront-Höhenkarten umfassen, die zu unterschiedlichen Zeiten erfasst werden, und das eine oder die mehreren Attribute eine zeitliche Variation zwischen den mehreren Wellenfront-Höhenkarten und eine lokalisierte räumliche Variation der mehreren Wellenfront-Höhenkarten umfassen.

5. Ophthalmisches System (100) nach Anspruch 1, wobei das Aberrometer (102) gemäß einem oder mehreren der Folgenden angeordnet ist:
(i) wobei das Aberrometer (102) dazu ausgelegt ist, den Brechungsfehler eines Auges (110) zu erfassen;
(ii) wobei das Aberrometer (102) dazu ausgelegt ist, Wellenfront-Aberrometrie zu implementieren, die die Ausbreitung einer Wellenfront durch das Auge (110) misst; wobei das Aberrometer (102) so konfiguriert ist, dass die Ausgabe des Aberrometers (102) eine Wellenfront-Höhenkarte ist, die ein zweidimensionales Array von Werten umfasst, wobei jeder Wert einen Punkt darstellt, von dem Licht gestreut wurde, und die Höhe an jedem Punkt die Phasenverzögerung einer Wellenfront bei Einfallen auf diesen Punkt darstellt.

6. Ophthalmisches System (100) nach Anspruch 1, ferner umfassend:
ein Lichtsdistanzierungs- und Entfernungsmessungssystem (LIDAR) (200), das mit dem Aberrometer (102) kombiniert und dazu ausgelegt ist, Tiefeninformationen gleichzeitig mit der einen oder den mehreren Wellenfront-Höhenkarten zu erfassen;
wobei das eine oder die mehreren Attribute die Tiefeninformationen umfassen.

7. Ophthalmisches System (100) nach Anspruch 6, wobei das eine oder die mehreren Attribute umfassen, ob die Tiefeninformationen Streuung innerhalb eines Glaskörpers (118) des Auges des Patienten (110) anzeigen.

8. Ophthalmisches System (100) nach Anspruch 6, wobei das Aberrometer (102) und das LIDAR-System (200) eine gemeinsame Laserlichtquelle (202) und einen gemeinsamen Scanspiegel (218) aufweisen.

9. Ophthalmisches System (100) nach Anspruch 1, wobei der ausführbare Code bei Ausführung durch die eine oder mehreren Verarbeitungsvorrichtungen (502) ferner die eine oder mehreren Verarbeitungsvorrichtungen dazu veranlasst, das eine oder die mehreren Attribute der einen oder mehreren Wellenfront-Höhenkarten, die den Glaskörpertrübungen (122) entsprechen, zu identifizieren, indem die eine oder mehreren Wellenfront-Höhenkarten mit einem Machine-Learning-Modell (518) verarbeitet werden.

## Revendications

1. Système ophtalmique (100) destiné au guidage de chirurgie ophtalmique comprenant :
un aberromètre (102) ;
un ou plusieurs dispositifs de traitement (502) ;
un ou plusieurs dispositifs de mémoire (508) couplés au ou aux dispositifs de traitement et stockant du code exécutable qui, lorsqu'il est exécuté par le ou les dispositifs de traitement, amène le ou les dispositifs de traitement à :
recevoir une ou plusieurs cartes d'élévation de front d'onde pour l'œil d'un patient (110) en provenance de l'aberromètre (102) ; **caractérisé en ce que** le code exécutable, lorsqu'il est exécuté par le ou les dispositifs de traitement, amène le ou les dispositifs de traitement à identifier un ou plusieurs attributs de la ou des cartes d'élévation de front d'onde correspondant aux flotteurs vitreux (122).

2. Système ophtalmique (100) selon la revendication 1, le ou les attributs comprenant une variation spatiale localisée de la ou des cartes d'élévation de front d'onde.

3. Système ophtalmique (100) selon la revendication 1, la ou les cartes d'élévation de front d'onde comprenant une pluralité de cartes d'élévation de front d'onde capturées à des instants différents et le ou les attributs comprenant une variation temporelle parmi la pluralité de cartes d'élévation de front d'onde.

4. Système ophtalmique (100) selon la revendication 1, la ou les cartes d'élévation de front d'onde comprenant une pluralité de cartes d'élévation de front d'onde capturées à des instants différents et le ou les attributs comprenant une variation temporelle parmi la pluralité de cartes d'élévation de front d'onde et une variation spatiale localisée de la pluralité de cartes d'élévation de front d'onde.

5. Système ophtalmique (100) selon la revendication 1, l'aberromètre (102) étant agencé selon un ou plusieurs des cas suivants :
(i) l'aberromètre (102) étant configuré pour détecter l'erreur de réfraction d'un œil (110) ;
(ii) l'aberromètre (102) étant configuré pour mettre en œuvre une aberrométrie de front d'onde qui mesure la propagation d'un front d'onde à travers l'œil (110) ;
l'aberromètre (102) étant configuré de telle sorte que la sortie de l'aberromètre (102) est une carte d'élévation de front d'onde comprenant un réseau bidimensionnel de valeurs où chaque valeur représente un point à partir duquel la lumière a été diffusée et l'élévation en chaque point représente le retard de phase d'un front d'onde lors de l'incidence sur ce point.

6. Système ophtalmique (100) selon la revendication 1, comprenant en outre :
un système de télédétection par laser, LIDAR (200) combiné avec l'aberromètre (102) et configuré pour capturer des informations de profondeur simultanément avec la ou les cartes d'élévation de front d'onde ;
le ou les attributs comprenant les informations de profondeur.

7. Système ophtalmique (100) selon la revendication 6, le ou les attributs comprenant si les informations de profondeur indiquent une diffusion à l'intérieur d'une humeur vitrée (118) de l'œil du patient (110).

8. Système ophtalmique (100) selon la revendication 6, l'aberromètre (102) et le système LIDAR (200) ayant une source de lumière laser commune (202) et un miroir de balayage commun (218).

9. Système ophtalmique (100) selon la revendication 1, le code exécutable qui, lorsqu'il est exécuté par le ou les dispositifs de traitement (502), amenant en outre le ou les dispositifs de traitement à identifier le ou les attributs de la ou des cartes d'élévation de front d'onde correspondant aux flotteurs vitreux (122) en traitant la ou les cartes d'élévation de front d'onde avec un modèle d'apprentissage machine (518).
